Europäisches Patentamt

European Patent Office

Office européen des brevets

(11) Publication number: **0 291 509 B1**

# EUROPEAN PATENT SPECIFICATION

(45) Date of publication of patent specification: **14.12.94** (51) Int. Cl.⁵: **A61M 37/00**

(21) Application number: **87901250.8**

(22) Date of filing: **02.02.87**

(86) International application number:
**PCT/US87/00200**

(87) International publication number:
**WO 87/04631 (13.08.87 87/18)**

(54) **SPRING DRIVEN INFUSION PUMP.**

(30) Priority: **03.02.86 US 825197**

(43) Date of publication of application:
**23.11.88 Bulletin  88/47**

(45) Publication of the grant of the patent:
**14.12.94 Bulletin  94/50**

(84) Designated Contracting States:
**AT BE CH DE FR GB IT LI LU NL SE**

(56) References cited:
**US-A- 3 731 681      US-A- 3 951 147
US-A- 4 056 095      US-A- 4 299 220
US-A- 4 447 224      US-A- 4 487 603
US-A- 4 557 726**

(73) Proprietor: **UNIVERSITY OF MINNESOTA
1919 University Avenue
St. Paul, MN 55104 (US)**

(72) Inventor: **DORMAN, Frank, D.
301 Burntside Drive
Minneapolis, MN 55422 (US)**
Inventor: **BUCHWALD, Henry
6808 Margarets Lane
Minneapolis, MN 55435 (US)**

(74) Representative: **Harrison, David Christopher
et al
MEWBURN ELLIS
York House
23 Kingsway
London WC2B 6HP (GB)**

## Description

Background of the Invention

The present invention relates to an implantable infusion pump for infusing drugs or other chemicals or solutions into a body therein the infusion pump is implanted. More particularly, the present invention relates to an implantable infusion pump which compensates for changes in ambient pressure and is largely unaffected by changes in ambient temperature so as to accurately control the flow rate of drugs from the implantable infusion pump into the body.

Infusion pump designs were rarely seen in medical literature until the 1950s. Most of these early infusion pumps were extracorporeal devices of various designs. One such device included a reciprocating air pump driven by an electric motor. Yet another design considered comprised a metal housing for a glass syringe and a compression chamber fed by a tank of nitrogen gas. Yet another such infusion pump included a motorized syringe pump which included an electric motor connected to the worm drive that moved a syringe plunge by a gear box. The gears were interchangeable such that replacement of the gears permitted different delivery rates. Yet another infusion pump included a syringe plunger driven by a rider on a threaded shaft. Numerous other designs were considered for these extracorporeal infusion pumps. P.D.W. Soden in his thesis entitled, "A Methodical Design Study of Miniature Profusion Devices For Chemotherapy of Cancer of the Head and Neck", studied possible designs for producing a miniature profusion device to be carried by ambulating patients receiving chemotherapeutic treatment for cancer of the head and neck. Quoting from his thesis, "Approximately two million alternative design solutions were synthesized and recorded in compact matrix form on a 'morphological chart'". One of the numerous design concepts mentioned by Soden for possible use with an extracorporeal infusion pump was the use of a small tubular arrangement containing an elastic metal bellows possibly constructed from preloaded disks so as to form a relatively small diaphragm in the tubular arrangement for exerting a fairly constant force on the drug solution being infused. Due to the size of the diaphragm, this design provided for very little, if any, compensation for changes in atmospheric pressure.

One of the earliest implantable infusion pumps intended for use in laboratory animals comprised a micro-injector comprising a compressed spring held away from a rubber-capped glass tube by a metal alloy disk with a low melting point. Administration of the injection was accomplished by placing the animal near the coils of a high-frequency induction heater. Activation of the coils melted the alloy disk and the spring ejected infusate into the desired site in the animal. A second implantable infusion pump for the continuous infusion of drugs utilized the osmotic pressure developed by a saturated aqueous solution of Congo red dye against water as its power source. The infusion pump comprised a partially collapsed rubber compartment filled with Congo red dye separated from a second water compartment by a semi-permeable cellophane member. Expansion of the rubber compartment as the water moved by osmosis into the Congo red solution ejected the drug from the infusion pump.

Implantable infusion pumps were clinically introduced in 1975. Implantable infusion pumps currently in clinical use or in animal trials anticipating clinical studies in the near future, include vapor pressure powered pumps, peristaltic pumps, and pulsatile solenoid pumps. The vapor pressure powered pump was developed at the University of Minnesota and is described hereafter. The peristaltic pump generally comprises a flexible tube placed in a u-shaped chamber in contact with rollers that press against the tube with sufficient force to occlude the tube's lumen. The rollers are rotated by a motor. As the rotor turns and the rollers compress the lumen of the tube, fluid is moved toward an exit. The rollers and housing are arranged so that a second roller begins to squeeze the tube before the first disengages, preventing backflow of the infusate. Sandia Laboratories, Siemens AG, and Medtronic, Inc. have developed implantable pumps with peristaltic pumping mechanisms. A pulsatile solenoid pump includes a solenoid driven reciprocating chamber with two check valves to move infusate from the reservoir out through the delivery catheter. Infusate is stored in a flexible metal diaphragm reservoir. Such a pump has been developed by Fischell and colleagues at Johns Hopkins University Applied Physics Laboratory and by the Pacesetter Corporation.

These currently available implantable infusion pumps provide drug infusion into the body at rates which are more precisely controllable than can be achieved by conventional oral and bolus injection methods. However, the existing implantable infusion pumps are sensitive to temperature and atmospheric pressure changes such that changes in temperature and atmospheric pressure cause corresponding changes in drug infusion rates from the implantable infusion pumps into the body. With some drugs, particularly those having small therapeutic indices, such changes in drug infusion rates are undesirable and, in certain situations, unacceptable.

One example of an existing implantable infusion pump is described in U.S. Patent No.

3.731,681, herein incorporated by reference, which describes an implantable infusion pump which uses a liquid/vapour equilibrium to maintain a constant pressure on a drug solution, such as insulin, contained in a drug chamber of the infusion pump in order to maintain a predetermined flow rate of the drug solution from the drug chamber via a capillary tube to an infusion site in the body. In the liquid/vapor powered pump, double chambered design with a rigid outer chamber and a flexible diaphragm separating the chambers is utilized. A liquid/vapor is present in one of the chambers either as a power source or to allow the diaphragm to move without creating a vacuum. However, due to the rigid outer shell structure of the pump, this technique of drug flow control is affected by changes in temperature and atmospheric pressure. Where the patient remains in a local region, the air pressure is a minor variable. However, there are conditions under which both temperature and pressure can change a significant amount. For example, if the patient has a fever, the temperature can change several degrees. The internal pressure change is about 0.5 psi per degree fahrenheit. Assuming a $54.9 \times 10^3$ Pa (8 psi) driving force at $37°C$ ($98.6°F$), a twenty-five percent (25%) increase in pressure and drug flow rate can result from a fever of $39.2°C$ (102.6 degrees fahrenheit). Such changes in flow rate may be unacceptable for certain drugs with small therapeutic indices.

An even more serious situation results from changes in atmospheric pressure. Atmospheric pressure change at any given location on the earth does not significantly affect flow rate of this pump. However, with modern modes of transportation, a patient can rapidly change altitude during travel, such as when traveling in the mountain or when travelling by plane wherein cabin pressures equivalent to 1524 to 1829m (five thousand to six thousand feet) of altitude are not uncommon. Since the vapor/pressure powered implantable infusion pump of U.S.-A-3,731,681 is enclosed in a rigid, immovable outer shell structure, it produces a constant internal pressure (at constant temperature) independent of the external pressure. The hydrostatic pressure within the body closely follows the external pressure on the body caused by atmospheric pressure. This is largely due to the compliance of the lungs and the venous circulation. The net effect is a pressure difference across the outflow resistance from the infusion pump (typically a capillary tube or the like) which changes linerally with external pressure. The drug flow rate can increase as much as forty percent (40%) when the patient takes a commercial airline trip.

One method of more accurately controlling the rate of drug delivery is an infusion regulator, such as that disclosed in U.S.-A-4,299,220. The infusion regulator described therein meters the rate of drug delivery on the basis of the pressure drop across the output or outflow resistance (capillary tube) using a diaphragm valve. A undesirable feature of the infusion regulator is that the drug solution flows through a metering valve at high local shear rates, which may be inappropriate for certain proteinaceous or micellar solutions.

US-A-4,487,603 discloses an implantable pump having a drug chamber with a flexible outer wall which tends to deform under the internal body pressure, and an electromagnetically-actuated pump assembly operated by batteries.

The present invention overcomes these and other problems associated with currently available implantable infusion pumps and infusion regulators.

## Summary of the Invention

The present invention relates to an infusion pump for implantation in a living body, comprising a housing having an external shell structure for defining a drug chamber and means for delivering a drug solution from the drug chamber to the living body, wherein said drug chamber has a variable volume defined by a flexible wall portion for exerting a force on the drug solution therein, the wall portion further forms a portion of said external shell structure and is subjected to a force exerted by internal body pressure, whereby changes in the internal body pressure cause corresponding changes of the force on the drug solution, wherein said flexible wall portion is formed by spring diaphragm means including a plurality of conical spring sections radially spaced apart and interconnected so as to maintain a substantially constant pressure differential on the drug solution to force the drug solution into the body via said delivery means.

In the preferred embodiment, the spring energy source means includes spring diaphragm means forming a flexible, exterior backwall of the drug chamber for applying pressure on the drug solution in the drug chamber, equal to a predetermined constant force exerted by the spring diaphragm plus force exe5ted by the internal body pressure.

The spring diaphragm means in one embodiment of the present invention preferably includes a spring diaphragm which exerts substantially constant force over a predetermined range of movement. The spring diaphragm forms a movable, flexible outer wall portion of the housing and cooperates with rigid wall portions of the housing to form a variable volume drug chamber. The internal pressure of the drug chamber is generated by the external spring diaphragm which is exposed to the internal pressure of the body at the implantation site. When the pump is implanted in soft tissue where it is not compressed by bony or other rigid structures, the external pressure acting on the spring diaphragm will not depart appreciably from atmospheric pressure. The internal pressure of the drug chamber will thus vary with external pressure exerted on the spring diaphragm and the pressure difference across the outlet conduit (e.g., capillary flow restrictor) will be substantially constant and correspond to the force applied on the drug chamber by the spring diaphragm.

Because the infusion pump of the present invention compensates for changes in ambient pressure, such a pump will perform properly when driven by internal pressures lower than those used in the vapor/pressure powered pump previously discussed, which uses a pressure of $54.9 \times 10^3$ - $68.6 \times 10^3$ Pa (8-10 psi) to minimize variation caused by temperature and atmospheric pressure changes. Reduction of this driving pressure in a vapor/pressure powered pump (e.g., by using a different gas) would increase error substantially. The spring driven infusion pump of the present invention can use a smaller operating pressure limited by different and smaller variables. The changes in external pressure on the pump and at the delivery site set a lower limit for the operating pressure of the present invention.

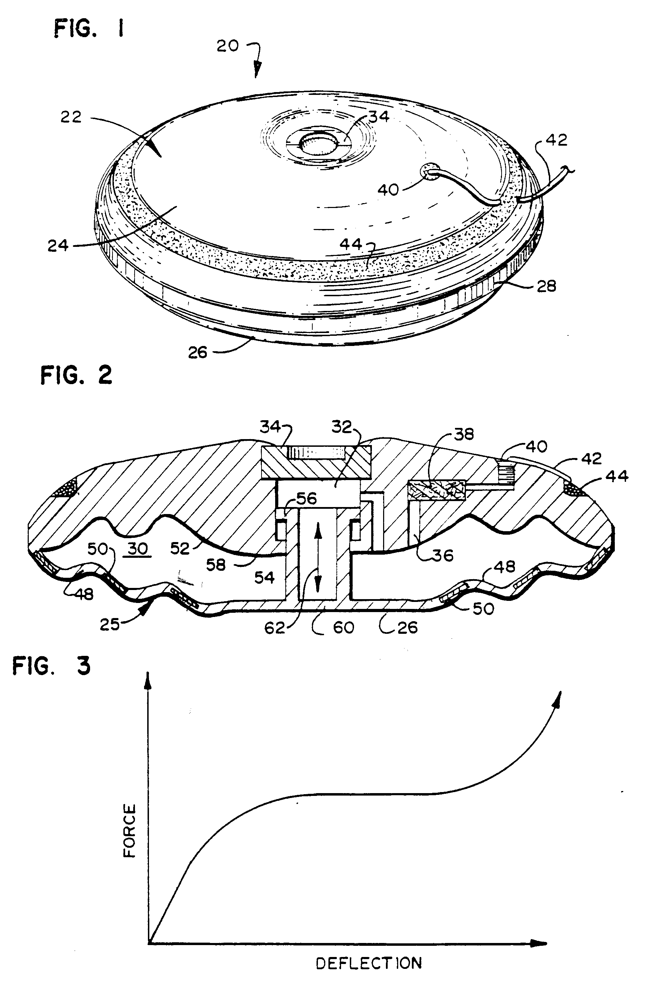

In the preferred embodiment of the present invention, the spring diaphragm forms a portion of the strong outer shell or housing of the infusion pump and is configured to provide a nearly constant force over the traveled distance corresponding to the infusion pump's drug delivery volume. Because of the relatively large area of the diaphragm, the force applied is large, thus requiring a relatively thick diaphragm. Constant force is obtained by using the snap action effect of conical washer springs. A plurality of conical spring sections are alternated radially with stronger reversed angle conical spring sections and/or substantially stiff cylindrical rings in order to make a substantially flat force/deflection curve. The proper thickness, cone angle, and material properties can be selected to give a constant force of a selected magnitude over a given distance, as illustrated in the force/deflection curve of Figure 3. Conical spring sections of this type are frequently referred to as Belleville washers and at a height to thickness ratio of 1.5:1, an extended linear force region can be obtained.

In the preferred embodiment, the inner surface of the top wall portion of the housing is configured so as to nest with the spring diaphragm so as to enable most of the drug solution contained in the drug chamber to be expelled. In some embodiments, the inner surface of the top wall portion might also include a spring diaphragm means. However, the primary flex action will occur at the thinner spring diaphragm forming a part of the bottom wall portion of the infusion pump.

These and various other advantages and features of novelty which characterize the present invention are painted out with particularity in the claims annexed hereto and forming a part hereof. However, for a better understanding of the invention, its advantages and objects attained by its use, reference should be had to the drawings which form a further part hereof and to the accompanying descriptive matter in which there is illustrated and described a preferred embodiment of the present invention.

Brief Description of the Drawings

In the drawings, in which like reference numerals and letters indicate corresponding parts throughout the several views;

Figure 1 is a view in perspective of an embodiment of an infusion pump in accordance with the principles of the present invention;

Figure 2 is a sectional view of the embodiment shown in Figure 1 with portions thereof being shown diagrammatically;

Figure 3 is a force/deflection curve illustrating substantially constant force over a predetermined range of deflection or movement exemplary of a spring diaphragm such as might be utilized in an infusion pump in accordance with the principles of the present invention;

Figure 4 is a sectional view of a single conical spring section;

Figure 5 is a diagrammatic view generally illustrating drug chamber pressure forces and movement of a flexible spring diaphragm into and out of a chamber generally in accordance with the principles of the present invention; and

Figure 6 is a sectional view of an infusion pump, operational elements thereof not being shown for purposes of illustration, illustrating a top inside surface thereof configured in accordance with the principles of the present invention to enable nesting of the flexible spring diaphragm thereagainst when the drug chamber is empty.

Detailed Description of a Preferred Embodiment of the Present Invention

Referring now to the drawings, there is illustrated in figures 1-2 a preferred embodiment of an implantable infusion pump in accordance with the principles of the present invention, the pump being generally designated by the reference numeral 20. The pump 20 has a housing 22 with top and bottom wall portions 24,26 interconnected by a side wall portion 28 forming a strong outer shell structure. (The expressions "top" and "bottom" are relative and refer only to positions that are generally shown in the drawings.) In the embodiment shown, the housing 22 has a generally cylindrical shape. The bottom wall portion 26 includes a flexible spring diaphragm 25 which cooperates with the remainder of the housing to define a variable volume, fluid-tight drug chamber 30 for holding a drug solution or other chemicals or solutions to be infused into an infusion site of a patient's body wherein the infusion pump is implanted.

As illustrated in Figures 1-2, the infusion pump 20 includes the standard features required of an implantable and refillable infusion pump. An inlet conduit 32 extends from the exterior of the housing 22 to the variable volume drug chamber 30 so as to provide for fluid communication from outside the housing 22 to the drug chamber 30. An upper end of the inlet conduit 32 includes a self-sealing, penetratable member or septum 34, suitably positioned therein in so as to provide a fluid type seal and yet provide for refilling of the drug chamber 30 by injection. An outlet conduit 36 leads from the drug chamber 30 to the exterior of the housing 22 so as to provide for outflow of drug solution from the drug chamber 30 to the exterior of the housing 22. The outlet conduit 36 is illustrated as including a suitable filter 38 for filtering out bacteria and trapped gas, which might be inadvertently introduced into the infusion pump 20 during the refilling process. Interconnected to an outer end of the outlet conduit 36 by a suitable connector 40 is capillary tubing 42 which serves as a flow regulating resistance element or flow restrictor. The capillary tubing 42 might be interconnected at an opposite end to a rubber catheter or the like that leads to the site of infusion in the body. Several metres of capillary tubing 42 is typically required (e.g 15-30m) (e.g., 50-100 feet).

The flow rate through the flow restrictor is governed by the Poisseuille equation as follows:

$Q = (\pi D^4 \Delta P)/128 \mu L$, where $Q$ = flow in ml/sec., $D$ = diameter in cm., $\mu$ = viscosity in poise, $\Delta P$ = pressure in dynes/cm$^2$, and $L$ = length in cm. The most readily adjustable parameters are the length and diameter of the capillary and the viscosity of the infusate. As illustrated, the capillary tubing 42 night be wrapped about the housing 22 in a groove 44 and suitably secured by a material compatible with body fluids. It will be appreciated that other types of devices might be used to provide for drug output or outflow resistance; for example, spiral groove plate, etched glass, steel capillary tubing, silica chip, etc. Moreover, the resistance elements may number more than one, as in the case of more than one site of infusion.

The outer surface of the top wall portion 24 of the housing 22 is preferably shaped to allow easy identification of the inlet conduit 32 and suitably protected with a layer of metal or the like to be protected from needle damage during the process of refilling the drug chamber 30. The bottom wall portion 26 and side wall portion 28 might also be similarly protected by a metal layer. It will be appreciated that the overall design of the infusion pump 20 can be more compact and have higher volumetric efficiency than vapor/pressure powered pumps since there is no second chamber and the outer shell structure of the infusion pump serves a dual purpose as the spring diaphragm and protective shell.

As with currently available implantable infusion pumps, the infusion pump 20 is constructed of materials non-toxic to the patient and compatible with both the drug solution and the body fluids. Titanium is a desirable material for forming a large portion of the housing 22, fittings, etc. All components of the infusion pump 20 will be made of materials compatible with body fluids and commonly used for construction of devices to be implanted within the body.

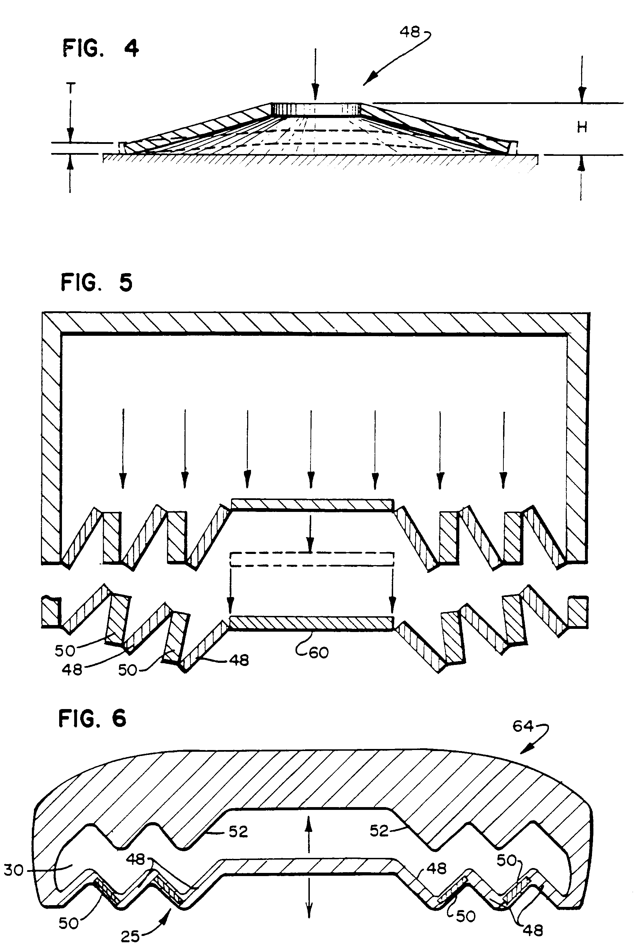

In the embodiment of the infusion pump shown in Figures 1-2, and as diagrammatically illustrated in Figure 5, the spring diaphragm 25 includes a series of nested conical sections 48 interconnected by stiff cylindrical ring sections 50 so as to form a substantially flat spring diaphragm. The conical sections 48 are constructed of an elastomer with a low elastic constant, and the ring sections 50 are preferably constructed of metal with a high elastic constant. The preferred construction technique is to mold the metal ring sections 50 into an elastomer structure forming the conical sections 48. If necessary, the inner surface of the spring diaphragm 25 can be coated with a plastic liner to resist drug action on the elastomer and reduce gas diffusion from the body into the drug chamber 30. A thin metal diaphragm might be used as a liner if necessary, to better isolate the drug solution in the drug chamber 30 by resisting gas and liquid diffusion.

The above described arrangement of conical sections 48 and ring sections 50 provide a spring diaphragm 25 with a longer useful range of movement or stroke than possible with a single conical spring section such as a single Belleville washer of the type that is generally shown in Figure 4. However, a Belleville washer such as shown in Figure 4 with the proper selection of cone angle and thickness can yield a force displacement curve as shown in Figure 3. The flat portion of the curve is a constant force region that can be used to produce a constant pressure over some range of displacement volume. The curve in Figure 3 is obtained when the ratio of height H of the cone to thickness T is about 1.5:1. If a strong material like Titanium is used, cone height must be very small, i.e., 10 to 20 thousandths of an inch, so as to provide force in the range suitable for infusion pumps such as 4 to 15 psi. This range of heights, which constitutes the effective stroke of a sprang diaphragm including a single conical spring, is too small to be practical for use in infusion pumps. In order to retain a flat pressure curve and achieve a longer stroke or range of movement of the spring diaphragm 25, a spring material with a lower elastic constant can be used; for example, plastics and elastomers. When low elastic materials are used, the thickness of the conical section can be increased and the cone angle made larger. This allows the spring diaphragm 25 to have a much longer range of travel in the substantially flat portion of the curve shown in Figure 3. The spring material also should have a much greater percent elongation in the elastic region of its stress strain curve. By separating the single conical spring into a nested series of conical sections interconnected by relatively stiff cylindrical ring sections a substantially flat spring diaphragm having an effective stroke or range of movement in the substantially flat portion of the force/deflection curve shown in Figure 3 which is required of infusion pumps is derived. Nesting of conical sections and movement of the spring diaphragm 25 is diagrammatically illustrated in Figure 5. In typical applications, the spring diaphragm will have a range of movement of stroke of about 1 to 2 cm. It will be appreciated that the shape and thickness of the spring diaphragm 25 may vary in order to exhibit the required force/deflection characteristics.

The conical sections are preferably made of a high temperature aerospace plastic like polyamide (Torlon™) or the aromatic polyester liquid crystal polymer (Xydor™). These materials have a flexual modulus of about two million psi versus about ten to twelve million psi for metals. Moreover, these plastics are moldable to the thicknesses and shapes required.

The spring diaphragm 25 forms the flexible bottom wall portion 26 of the drug chamber 30 as generally shown in Figure 2. The outside surface of the flexible spring diaphragm 25 is exposed to the body and senses internal body pressure so as to compensate for changes in the internal body pressure caused by changes in atmospheric pressure and temperature. The flexible spring diaphragm 25 communicates the internal body pressure to the drug chamber 30. In the embodiment shown in Figure 2, and as illustrated in Figure 6, an inner surface 52 of the top wall portion 24 is preferably configured, i.e., has a somewhat convoluted shape, so as to allow the spring diaphragm 25 to nest into the complimentary shape of the inner surface 52. This enables the spring diaphragm 25 to expel substantially all of the drug solution from the drug chamber 30 prior to refilling of the drug chamber 30. Moreover, the inner surface 52, just as with the spring diaphragm 25, might include a nested series of conical sections interconnected by a substantially stiff cylindrical ring in order to provide a spring diaphragm complementary to than of the spring diaphragm 25. It will be appreciated, that in this embodiment, the spring diaphragm 25 of the bottom wall portion 26 will provide most of the flexing action.

The spring diaphragm 25 is extended beyond its nested position when assembled such that the spring diaphragm 25 is therefore under stress. The initial displacement is selected to bring the pressure or force exerted by the spring diaphragm 25 to the flat portion of the force/displacement curve illustrated in Figure 3. The functional volume of the infusion pump 20 is that displacement which takes place over this substantially flat region of the force/deflection curve. To limit the filling of the infusion pump to this displacement of the spring diaphragm 25, a telescoping section 54 is interconnected to the spring diaphragm 25 and extends into the inlet conduit 32. When the telescoping section 54 is fully extended, collar portion 56 cooperates with a collar portion 58 of the inlet conduit 32 to prevent the spring diaphragm 25 from traveling more than the desired distance. As illustrated, the telescoping section 54 is interconnected to a substantially flat portion 60 of the spring diaphragm. The telescoping section 54, thus limits the stroke of the spring diaphragm 25 as indicated generally by the arrows 62 and causes the filling back pressure to increase rapidly, thereby, reducing the risk of damaging the spring diaphragm 25 or causing errors in a drug flow rate due to excess pressure in the drug chamber.

It will be appreciated that the drug infusion site must be considered in the design of the infusion pump. For example, if the catheter must deliver the drug into the relatively high pressure of the arterial system, the pump pressure will need to be larger to maintain the same error limits that can be obtained when delivering the drug intravenously or intraperitoneally.

Moreover, although a preferred embodiment of the present invention has been described above, it will be appreciated that other pressure compensating means in accordance with the principles of the present invention might be utilized. In particular, other constant force spring arrangements might be utilized as a pressure source.

It is to be understood that even though the above numerous characteristics and advantages of the invention have been set forth in the foregoing description, together with details of the structure and function of the invention, the disclosure is illustrative only, and changes may be made in detail, especially in matters of shape, size and arrangement of parts.

## Claims

1. An infusion pump for implantation in a living body, comprising:

   a housing (22) defining a drug chamber (30); and

   means (36,38,40,42) for delivering a drug solution from the drug chamber (30) to the living body;

   wherein said drug chamber (30) has a variable volume defined by a flexible wall portion (25) for exerting a force on the drug solution therein, the wall portion (25) further forms a portion of said Housing (22) to be subjected to a force exerted by pressure in the living body, whereby changes in the internal body pressure cause corresponding changes of the force on the drug solution;

   characterised in that

   said flexible wall portion (25) is formed by spring diaphragm means including a conical spring member (48,50) to maintain a substantially constant pressure differential on the drug solution to force the drug solution into the body via said delivering means.

2. An infusion pump in accordance with claim 1, wherein the spring diaphragm means exerts a pressure of $2.7 \times 10^4$ to $10.3 \times 10^4$ Pa (4 to 5 psi) on the drug solution in the drug chamber.

3. An infusion pump in accordance with claim 1 or claim 2 wherein the infusion pump includes means (54) for limiting movement of the spring diaphragm means to a range of movement so as to maintain said substantially constant pressure differential.

4. An infusion pump in accordance with claim 3, wherein the spring diaphragm means is movable between a first position and a second position defined by the limiting means, the spring diaphragm means being stressed at both positions so as to maintain said substantially constant pressure differential over said range of movement between the first and second positions.

5. An infusion pump in accordance with any preceding claim, wherein the spring diaphragm means is the principal force means for forcing the drug solution out of the drug chamber.

6. An infusion pump in accordance with any preceding claim wherein the means for delivering the drug solution to the body from the drug chamber include outlet conduit means for providing fluid communication between the drug chamber and an infusion site in the body, the outlet conduit means including flow resistance means (42) for resisting drug solution flow.

7. An infusion pump in accordance with any preceding claim, further comprising inlet conduit means (32) for providing fluid communication between the drug chamber and an exterior of the housing.

8. An infusion pump in accordance with claim 7 including a self-sealing, penetrable member (34) in the inlet conduit means (32), the self-sealing, penetrable member being unobstructed so that the pump can be implanted in the body with the unobstructed penetrable member situated adjacent a surface area of the body whereby the drug chamber can be refilled with drug solution periodically by injection through the skin.

9. An infusion pump in accordance with any one of the preceding claims wherein the conical spring member is a plurality of conical spring sections (48,50) spaced apart and interconnected so as to maintain the said force.

10. An infusion pump in accordance with claim 9, wherein the plurality of conical spring sections (48,50) are radially interconnected by sections (50,48) having a higher elastic constant.

11. An infusion pump in accordance with claim 10, wherein the plurality of conical spring sections (48) and the sections (50) having a higher elastic constant are alternated radially, the latter sections (50) being reversely oriented to the conical spring sections.

## Patentansprüche

1. Infusionspumpe zur Implantierung in einen lebenden Körper, umfassend:

   ein Gehäuse (22), das eine Arzneimittelkammer (30) definiert;

   und

   Mittel (36, 38, 40, 42) zum Abgeben einer Arzneimittellösung von der Arzneimittelkammer (30) an den lebenden Körper;

   worin die genannte Arzneimittelkammer (30) ein durch einen flexiblen Wandabschnitt (25) definiertes variables Volumen zum Anwenden einer Kraft auf die Arzneimittellösung darin aufweist, der Wandabschnitt (25) weiters einen Abschnitt des genannten Gehäuses (22) bildet, das einer durch den Druck im lebenden Körper ausgeübten Kraft ausgesetzt werden soll, wodurch Veränderungen im inneren Körperdruck entsprechende Veränderungen der Kraft auf die Arzneimittellösung bewirken;
dadurch gekennzeichnet, daß
der genannte flexible Wandabschnitt (25) durch Federdiaphragmamittel einschließlich eines konischen Federmittels (48, 50) gebildet wird, um ein im wesentlichen konstantes Druckdifferential auf die Arzneimittellösung aufrechtzuerhalten, um die Arzneimittellösung über die genannten Abgabemittel mit Kraft in den Körper zu pressen.

2. Infusionspumpe nach Anspruch 1, worin das Federdiaphragmamittel einen Druck von 2,7 x $10^4$ bis 10,3 x $10^4$ Pa (4 bis 5 psi) auf die Arzneimittellösung in der Arzneimittelkammer ausübt.

3. Infusionspumpe nach Anspruch 1 oder Anspruch 2, worin die Infusionspumpe Mittel (54) zum Begrenzen der Bewegung des Federdiaphragmamittels auf einen Bewegungsbereich enthält, um das genannte im wesentlichen konstante Druckdifferential aufrechtzuerhalten.

4. Infusionspumpe nach Anspruch 3, worin das Federdiaphragmamittel zwischen einer ersten und einer zweiten, durch das Begrenzungsmittel definierte Position bewegbar ist, wobei das Federdiaphragmamittel an beiden Positionen gespannt ist, um das genannte im wesentlichen konstante Druckdifferential im genannten Bewegungsbereich zwischen der ersten und zweiten Position aufrechtzuerhalten.

5. Infusionspumpe nach einem der vorhergehenden Ansprüche, worin das Federdiaphragmamittel das hauptsächliche Kraftmittel zum Befördern der Arzneimittellösung aus der Arzneimittelkammer ist.

6. Infusionspumpe nach einem der vorhergehenden Ansprüche, worin die Mittel zum Abgeben der Arzneimittellösung von der Arzneimittelkammer an den Körper Auslaßleitungsmittel zum Schaffen von Fluidkommunikation zwischen der Arzneimittelkammer und einer Infusionsstelle im Körper enthalten, wobei die Auslaßleitungsmittel Flußwiderstandsmittel (42) enthalten, um dem Arzneimittellösungsfluß entgegenzuwirken.

7. Infusionspumpe nach einem der vorhergehenden Ansprüche, weiters umfassend Einlaßleitungsgsmittel (32) zum Schaffen von Fluidkommunikation zwischen der Arzneimittelkammer und einem Äußeren des Gehäuses.

8. Infusionspumpe nach Anspruch 7, umfassend ein selbstabdichtendes, durchstechbares Element (34) im Einlaßleitungsmittel (32), wobei das selbstabdichtende, durchstechbare Element nicht blockiert ist, sodaß die Pumpe im Körper implantiert werden kann, wobei das nicht blockierte, durchstechbare Element angrenzend zu einem Oberflächenbereich des Körpers angeordnet ist, wodurch die Arzneimittelkammer durch eine Injektion durch die Haut in periodischen Abständen mit Arzneimittellösung nachgefüllt werden kann.

9. Infusionspumpe nach einem der vorhergehenden Ansprüche, worin das konische Federmittel aus einer Vielzahl an konischen Federabschnitten (48, 50) besteht, die voneinander beabstandet und miteinander verbunden sind, um die genannte Kraft aufrechtzuerhalten.

10. Infusionspumpe nach Anspruch 9, worin die Vielzahl an konischen Federabschnitten (48, 50) durch Abschnitte (50, 48) mit einer höheren Elastizitätskonstanten radial miteinander verbunden ist.

11. Infusionspumpe nach Anspruch 10, worin die Vielzahl an konischen Federabschnitten (48) und die Abschnitte (50) mit einer höheren Elastizitätskonstanten einander radial abwechseln, wobei die letzteren Abschnitte (50) umgekehrt zu den konischen Federabschnitten ausgerichtet sind.

## Revendications

1. Pompe d'infusion à implanter dans un corps vivant, comprenant :
un boîtier (22) définissant une chambre à médicament (30); et
des moyens (36, 38, 40, 42) pour délivrer une solution de médicament à partir de la chambre à médicament (30) vers le corps vivant;
dans laquelle ladite chambre à médicament (30) a un volume variable défini par une partie de paroi flexible (25) pour exercer une force sur la solution de médicament dans celle-ci, la partie de paroi (25) formant en outre une partie dudit boîtier (22) destiné à subir une force exercée par la pression dans le corps vivant, de sorte que des variations dans la pression interne du corps engendre des variations correspondantes de la force sur la solution de médicament ; caractérisée en ce que
ladite partie de paroi flexible (25) est formée de moyens formant membrane élastique comprenant un élément formant ressort conique (48, 50) pour maintenir un différentiel de pression sensiblement constant sur la solution de médicament pour forcer la solution de médicament dans le corps par l'intermédiaire desdits moyens de délivrance.

2. Pompe d'infusion selon la revendication 1, dans laquelle les moyens formant membrane élastique exercent une pression comprise entre $2,7 \times 10^4$ et $10,3 \times 10^4$ Pa (4 à 5 psi) sur la solution de médicament dans la chambre à médicament.

3. Pompe d'infusion selon la revendication 1 ou 2, dans laquelle la pompe d'infusion comprend un moyen (54) pour limiter les mouvements des moyens formant membrane élastique dans une plage de mouvement de manière à maintenir ledit différentiel de pression sensiblement constant.

4. Pompe d'infusion selon la revendication 3, dans laquelle les moyens formant membrane élastique sont mobiles entre une première position et une seconde position définies par le moyen de limitation, les moyens formant membrane élastique étant contraints dans les deux positions de manière à maintenir ledit différentiel de pression sensiblement constant sur toute ladite plage de mouvement entre les première et seconde positions.

5. Pompe d'infusion selon l'une quelconque des revendications précédentes, dans laquelle les moyens formant membrane élastique sont les moyens de force principaux pour forcer la solution de médicament hors de la chambre à médicament.

6. Pompe d'infusion selon l'une quelconque des revendications précédentes, dans laquelle les moyens pour délivrer la solution de médicament au corps à partir de la chambre à médicament comprennent un moyen formant conduit d'évacuation pour produire une communication de fluide entre la chambre à médicament et un site d'infusion dans le corps, le moyen formant conduit d'évacuation comprenant un moyen de résistance à l'écoulement (42) pour résister à l'écoulement de la solution de médicament.

7. Pompe d'infusion selon l'une quelconque des revendications précédentes, comprenant en outre un moyen formant conduit d'admission (32) pour produire une communication de fluide entre la chambre à médicament et l'extérieur du boîtier.

8. Pompe d'infusion selon la revendication 7, comprenant un élément pénétrable auto-étanchéifiant (34) dans le moyen formant conduit d'admission (32), l'élément pénétrable autoétanchéifiant étant non obstrué de sorte que la pompe peut être implantée dans le corps avec l'élément pénétrable non obstrué situé adjacent à une surface du corps de sorte que la chambre à médicament peut être rechargée avec une solution de médicament périodiquement par injection à travers la peau.

9. Pompe d'infusion selon l'une quelconque des revendications précédentes, dans laquelle l'élément formant ressort conique est une pluralité de tronçons de ressort conique (48, 50) espacés les uns des autres et interconnectés de manière à maintenir ladite force.

10. Pompe d'infusion selon la revendication 9, dans laquelle la pluralité de tronçons de ressort conique (48, 50) sont radialement interconnectés par des tronçons (50, 48) ayant une raideur élastique plus élevée.

11. Pompe d'infusion selon la revendication 10, dans laquelle la pluralité de tronçons de ressort conique (48) et les tronçons (50) ayant une raideur élastique plus élevée sont alternés radialement, les derniers tronçons (50) étant inversement orientés par rapport aux tronçons de ressort conique.

FIG. 1

FIG. 2

FIG. 3

FORCE

DEFLECTION

FIG. 4

FIG. 5

FIG. 6